# EUROPEAN PATENT SPECIFICATION

(11) **EP 1 529 515 B1**
(45) Date of publication and mention of the grant of the patent: **31.12.2008**
(21) Application number: 03025174.8
(22) Date of filing: 04.11.2003
(51) Int. Cl.: A61K 8/34, A61Q 5/06

(54) **Hair styling composition**
Haarstylingzusammensetzung
Composition de coiffage

(43) Date of publication of application: 11.05.2005
(73) Proprietor: KPSS-Kao Professional Salon Services GmbH, 64297 Darmstadt (DE)
(72) Inventor: Cajan, Christine, 56130 Bad Ems (DE); Lehn, Jutta, 64285 Darmstadt (DE)

(56) References cited:
- EP-A- 0 756 859
- EP-A- 1 060 733
- EP-A- 1 283 030
- EP-A- 1 384 404
- WO-A-99/39683
- US-A1- 2002 146 379
- US-A1- 2003 035 783
- US-B1- 6 503 517
- "International Cosmetic Ingredient Dictionary" 1995, THE COSMETIC,TOILETRY, AND FRAGRANCE ASSOCIATION "Carbomer"

## Description

This invention relates to a hair styling composition with cooling effect on scalp when applied onto hair and scalp, The compositions of the present invention makes as well re-styling of hair possible after humidifying and/or wetting. Compositions can as well be used suitably for hair caring purposes.

Compositions have been known for conditioning and/or styling hair for some time. They customarily comprise in an aqueous and/or aqueous-alcoholic medium hair conditioning substances such as cationic polymers, cationic surfactants and as well hair styling polymers of anionic, cationic, non-ionic and/or amphoteric or zwitterionic character.

Although state of the art is developed to a certain extent there is still need for improvements in hair styling area in particular in hair restyling, achieving new style, without washing hair and without any need for new styling products and as well improvement in scalp feelings with the application of products designed for hair-styling. It should here be noted that within the range of the state of the art, attention is always paid to hair- styling, and so far improvements in scalp feelings at the same time is not addressed at all.

For example in EP 756 859, a styling composition is disclosed comprising grafted silicone polymer and two additional polymers of different ionic characters selected from group of anionic, cationic, non-ionic or amphoteric ones. In the whole length of the patent specifications cooling effect of such composition on scalp has not been touched at all.

EP-1283030, WO-99/39683, EP-106073, US-2002/146377 and US-2003/035783 disclose hair treating compositions comprising menthol.

The subject of the present invention is an aqueous and/or aqueous-alcoholic hair-styling composition according to claim 1 comprising a styling polymer and a second polymer and at least one cooling agent selected from menthol and/or menthyl lactate. The compositions of the present invention can be formulated as an aerosol hair and scalp sprays and as well as solutions to be sprayed onto hair and scalp using a mechanical pump device.

Upon application of the compositions of present invention, hair properties can as well be improved such as manageability, shine, volume and body and elasticity.

For a successful hair styling process, sufficient amount of composition of the present invention must be applied onto a freshly washed and/or shampooed hair.

Composition of the present invention makes as well hair re-styling possible, without washing hair freshly and without use of new styling products. In the restyling process, on already washed, optionally shampooed hair and styled primarily with the compositions of the present invention, firstly water and/or water-alcohol mixture is applied, either by spraying or pouring in portions onto hair, for humidifying and/or wetting the hair. Subsequently styling can be carried out with use of those obvious brush and additional physical aids as in the case of styling hair freshly. By doing so new styles can easily be achieved without a new hair wash and need for additional styling products.

The cooling agents are selected from the well known ones menthol or mentyl lactate or their mixtures. Certainly, the used solvents such as alcohol have also been proven to show cooling effect on skin. According to the present invention it has been found out that in the presence of one or more above-mentioned cooling agents, perceivable effect is understood to be much better and/or improved. The total concentration of the cooling agents, menthol or menthyl lactate or their mixtures, is in the range of 0.05 to 1.5%, preferably 0.05 to 1%, more preferably 0.05 to 0.75% by weight calculated to the total weight of the composition.

The styling polymers are selected from the anionic, non-ionic, cationic and/or amphoteric or zwitterionic ones. The styling composition of the present invention comprises a styling polymer and a second polymer. Certainly, the similar effects are achieved when more than one polymer of the same type or as well of different types in combination are used.

Non-ionic polymers are selected from the ones soluble in water and/or alcohol and/or in alcohol water mixtures, at any ratio. Under the definition of soluble in alcohol and alcohol water mixture, it should be understood that the polymer is soluble in lower alcohols such as ethanol, n-propanol or isopropanol and in their mixtures with water, at any ratio

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64 from BASF AG.

Further non-ionic polymer suitable for compositions of the present invention is vinylpyrrolidone/vinylacetae/vinylpropionate copolymer known with the trade name Luviskol VAP 343 as well from BASF.

Natural non-ionic polymers are as well suitable for the composition of the present invention. Those are such as cellulose, chitosan, guar gum, neutralised shellac and their derivatives.

Cationic polymers are of greater variability in their structure found to be suitable for the styling composition of the present invention. It has been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quatemium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quatemium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers

The cationic polymers also include the quaternized products of graft polymers from organopolysiloxanes and polyethyl oxazolines described in EP-A 524 612 and EP-A 640 643.

Amphoteric or zwitterionic polymers, preferably be used in mixture with at least one non-ionic and/or cationic polymers, are also found to be suitable for styling composition of the present invention. Examples to the preferred ones are copolymerisate of n-octylacrylamide, acrylic or methacrylic acid and tert.-butylaminoethylmethacrylate known with its trade name Amphomer, copolymer of methacryloylethylbetaine and alkyl methacrylate known as Yukaformer, terpolymer of methacrylic or acrylic acid and itaconoic acid and a basic monomer of mono or dialkylaminoalkyl acrylate or methacrylate or acryla of methacrylamide known with the trade name Aquaflex SF 40.

As amphoteric polymers which can be used alone or in mixture with at least one additional cationic and/or nonionic polymer, special reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer@", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Anionic polymers are as well suitable for styling compositions of the present invention. Suitable ones are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Concentration of styling polymers of anionic, cationic, non-ionic and/or amphoteric or zwitterionic character is in the range of 0.05 - 10%, preferably 0.05 - 7.5% and most preferably 0.05 - 5% by weight, calculated to the total composition.

The styling composition of present invention can comprise hair-conditioning agents. Conditioning agents can be selected from oily substances, non-ionic substances, cationic amphiphilic ingredients, cationic polymers (as mentioned above) or their mixtures. Oily substances are selected from such as silicone oils, either volatile or non-volatile, natural and synthetic oils. Among silicone oils those can be added to the pre-treatment composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil.

Non-ionic conditioning agents can as well be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II, respectively,

R₁ CO (O CH₂ CH₂)ₙ OH formula I

R₁ CO (O CH₂ CH₂)ₙ O OC R₂ formula II

where R₁ and R₂ are independent from each other saturated, unsaturated or branched or non-branched alkyl chain with 7 to 21 C atoms and n is typically 2 -100.

Hair styling composition can contain further cationic amphiphilic conditioning ingredients according to the formula III. 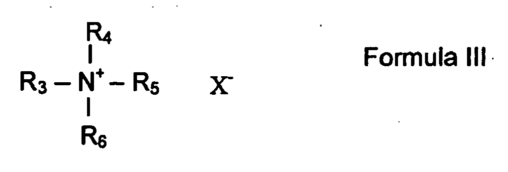 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 1 - 4 or

R₈ CO O (CH₂)ₙ

where R₈ is saturated or unsaturated, branched or non-branched alkyl chain with 7-21 C atoms and n has typical value of 1 - 4, and
R₄ is H or unsaturated or saturated, branched or non-branched alkyl chain with 1 - 22 C atoms or

R₇ CO NH (CH₂)ₙ

or

R₈ CO O (CH₂)ₙ

where R₇, R₈ and n are same as above.

R₅ and R₆ are H or lower alkyl chain with 1 to 4 Carbon atoms, and X is typically chloride, bromide or methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate. Those can at the same time serve as solubilizing agents for those ingredients difficult to integrate into the formulations.

The cationic polymers mentioned above used for achieving styling are as well found to be suitable for hair conditioning purposes when formulated into the compositions of the present invention.

Typical concentration range for any of the conditioners mentioned above can be 0.01 - 5% by weight, preferably 0.01 - 3.5% by weight and more preferably 0.05 - 2.5% by weight calculated to the total composition.

Solubilizers of anionic, nonionic, cationic and amphoteric character can as well be used in combination in the conditioners of the present invention. In this case, the total concentration of the solubilizer in the final product should, as a rule, not exceed 2%, prefereably 1.5% and more preferably 1.0% by weight calculated to the total composition. It should be noted that the solubilizers preferred are nonionic and cationic surfactants.

Any of the cationic surfactant mentioned above can serve as well as solubilizer. For solubilizing purpose the preferred ones are those with single alkyl chain such as cetrimonium chlorise, steratrimonium chloride.

The non-ionic surfactants are found to be particularly suitable as solubilizer. Those ethoxylates of hydrogenated castor oil such as PEG 40 hydrogenated castor oil are found to be particularly suitable know with the trade name Cremophor from BASF.

Other suitable non-ionic and anionic and amphoteric or zwitterionic surfactants can be found in the monograph of Schrader, K. H., Grundlagen und Rezepturen der Kosmetika, pages 681 - 695.

The concentration of solubilizers in the formulation should not exceed 2%, preferably 1.5% most preferably 1% by weight, calculated to the total composition.

The compositions according to the invention can comprise propellant and/or mixtures of propellants, preferably in an amount up to 50% by weight, calculated to the total aerosol foam composition, in the case that an aerosol prepartions is produced.

Useful propellants are, for example, hydrocarbons such as propane, n-butane, i-butane and the mixtures thereof, dimethyl ether, and/or carbon dioxide. The pressure in the aerosol container preferably ranges from about 1.5 to 6 bar.

Moreover, the hair styling compositions according to the invention can comprise all compounds customarily found in hair styling and/or conditioning compositions, such as perfumes, dyestuffs both for coloration of the products, as well as direct-acting dyestuffs for the coloration of human hair in accordingly increased concentrations, solvents and solubilizers, pH-regulants, preservatives, vitamins, plant extracts, neutralizing agents for the incorporated polymers, etc., in suitable, known amounts.

The pH-value of the compositions according to the invention is not critical and can basically vary between about 2 and 8.5, in particular about 3 and 8.

The following examples are to illustrate the invention, but not limiting it.

### Example 1

### Pumpspray

| | % |
|---|---|
| Ethanol | 90.0 |
| Polyquaternium-11 | 0.2 |
| Polyvinylcaprolactam | 1.0 |
| Menthyl lactate | 0.3 |
| Menthol | 0.3 |
| Fragrance | 0.2 |
| PEG-40 Hydrogenated Castoroil | 0.2 |
| Water | add 100.0 |

Polymers are dissolved in ethanol, mentyl lactate, methnol, and fragrance is dissolved/mixed with PEG-40 hydrogenated castor oil and than added to the solution of polymers in ethanol. Finally, the composition is made up to 100% by adding water.

### Example 2 (Reference example)

### Aerosolspray

| | |
|---|---|
| Ethanol | 35.0 |
| PVP (Luviskol K 90) | 0.2 |
| Menthyl Lactate | 0.3 |
| Fragrance | 0.2 |
| PEG 40 Hydrogenated Castoroil | 0.2 |
| Water | 34.1 |
| Dimethylether | 30.0 |

The composition is prepared in the same way as in Example 1 except that the propellant, dimethylether (DME), is added after transferring the solution into its final vessel.

### Example 3

### Pumpspray

| | |
|---|---|
| PVP (Luviskol K 90) | 0.3 |
| Acrylates/Octylacrylamide Copolymer | 1.0 |
| Aminomethyl Propanol | 0.25 |
| Fragrance | 0.2 |
| Menthol | 0.3 |
| Ethanol | add 100 |

The composition is prepared in the same way as in Example 1.

### Example 4

### Pumpspray

| | % |
|---|---|
| Polyquaternium-11 | 0.25 |
| VPNA Copolymer | 2.0 |
| Menthol | 0.2 |
| Menthyllactate | 0.2 |
| Ethanlol | 40 |
| Fragrance | 0.2 |
| PEG-40 Hydrogenatet Casatoroil | 0.2 |
| Water | add 100 |

The composition is prepared in the same way as in Example 1.

### Example 5

### Pumpspray

| | % |
|---|---|
| Shellac (neutralized) | 1.0 |
| PVP | 0.2 |
| Menthol | 0.3 |
| Fragrance | 0.2 |
| Ethanol | add 100 |

The composition is prepared in the same way as in Example 1.

### Example 6

### Pumpspray

| | % |
|---|---|
| Chitosan | 0.1 |
| VP/VA Copolymer | 2.0 |
| Menthyl lactate | 0.5 |
| Fragrance | 0.2 |
| PEG-40 Hydrogenated castor oil | 0.2 |
| Ethanol | 10 |
| Water | add 100 % |

The composition is prepared in the same way as in Example 1.

## Claims

1. Aqueous and/or aquesous-alcoholic hair styling composition with cooling effect on scalp **characterised in that** it comprises a hair styling polymer selected from non-ionic, anionic, cationic and/or amphoteric or zwitterionic ones and at least one cooling agent selected from menthol and menthyllactate wherein a composition consisting of
| | |
|---|---|
| Carbopol 934 | 0.50 |
| Polyquaternium-10 | 0,05 |
| Ethanol (denatured) | 25.00 |
| PEG-60 hydrogenated castor oil | 0.15 |
| Ceteareth-20 | 0.15 |
| Natural Menthol | 0.5 |
| Natural Camphor | 0.05 |
| Salicylic acid | 0.01 |
| Oligochitosan | 1.00 |
| Niacinamide | 0,05 |
| Vitamine E | 0.05 |
| Hydrolyzed collagen | 0.01 |
| Panthenol | 0.05 |
| Panthenyl ethyl ether | 0.05 |
| Octyl methoxycinnamate | 0.09 |
| Benzophenone-3 | 0.09 |
| dl-alphatocopherol acetate | 0,03 |
| Lactic acid | 0.05 |
| Perfume | 0.35 |
| Triethanolamine | 0.40 |
| Dimethicone | 0.05 |
| 2-phenyl propyl MQ resin | 0.5 |
| Water and minos | q.s. to 100 |
is excluded from the scope. and a second polymer as well selected from non-ionic, anionic, cationic and/or amphoteric or zwifterionic ones

2. Hair styling composition according to claim 1 **characterised in that** it comprises at least one propellant gas.

3. Hair styling composition according to any of the preceding claims **characterised in that** it comprises hair styling polymers at a concentration of 0.05 - 10% by weight, calculated to the total composition.

4. Hair styling composition according to any of the preceding claims **characterised in that** it comprises cooling agent at a concentration of 0.05 - 2% by weight, calculated to the total composition.

5. Hair styling composition according to any of the preceding claims **characterised in that** it comprises hair conditioning agents at a concentration of 0.05 - 5% by weight calculated to the total composition.

6. Hair styling composition according to any of the preceding claims **characterised in that** it comprises anionic, non-ionic, cationic and/or amphoteric or zwitterionic surfactants as solubilizers.

7. Process for styling hair **characterised in that** on freshly washed hair sufficient amount of the composition according to any of the preceding claims is applied.

8. Process for re-styling hair styled as in claim 7 **characterised in that** new style is achieved after humidifying and/or wetting said hair by spraying and/or pouring water and/or water alcohol mixture at any ratio without freshly washing and/or shampooing hair and without need for new styling product.

## Patentansprüche

1. Wässrige und/oder wässrig-alkoholische Haarstyling-Zusammensetzung mit Kühleffekt auf der Kopfhaut, **dadurch gekennzeichnet, dass** sie ein Haarstyling-Polymer, ausgewählt aus nichtionischen, anionischen, kationischen und/oder amphoterischen oder zwitterionischen Polymeren, und ein zweites Polymer, ebenfalls ausgewählt aus nichtionischen, anionischen, kationischen und/oder amphoterischen oder zwitterionischen Polymeren, und mindestens einen kühlenden Wirkstoff, ausgewählt aus Menthol und Menthyllactat, enthält, wobei eine Zusammensetzung bestehend aus
| | |
|---|---|
| Carbopol 934 | 0.50 |
| Polyquaternium-10 | 0.05 |
| Ethynol (denaturiert) | 25.00 |
| PEG-60 hydrogenisiertes Rizinusöl | 0.15 |
| Ceteareth-20 | 0.15 |
| natürlichesl Menthol | 0.5 |
| natürlicher Kampfer | 0.05 |
| Salicylsäure | 0.01 |
| Oligochitosan | 1.00 |
| Niacinamide | 0.05 |
| Vitamin E | 0.05 |
| Hydrolysiertes Collagen | 0.01 |
| Panthenol | 0.05 |
| Panthenyl ethyl ether | 0.05 |
| Octyl methoxycinnamate | 0.09 |
| Benzophenone-3 | 0.09 |
| dl-alphatocopherol acetate | 0.03 |
| Milchsäure | 0.05 |
| Parfüm | 0.35 |
| Triethanolamine | 0.40 |
| Dimethicone | 0.05 |
| 2-phenyl propyl MQ resin | 0.5 |
| Wasser und weniger bedeutende Elemente | q.s. auf 100. |
aus dem Schutzbereich ausgenommen ist.

2. Haarstyling-Zusammensetzung nach Anspruch 1, **dadurch gekennzeichnet, dass** sie mindestens ein Treibgas enthält.

3. Haarstyling-Zusammensetzung nach einem der vorhergehenden Ansprüche, **dadurch gekennzeichnet, dass** sie Haarstyling-Polymere in einer Menge von 0,05 - 10 Gew.- %, berechnet auf die Gesamtzusammensetzung, enthält.

4. Haarstyling-Zusammensetzung nach einem der vorhergehenden Ansprüche, **dadurch gekennzeichnet, dass** sie einen kühlenden Wirkstoff in einer Menge von 0,05 - 2 Gew.- %, berechnet auf die Gesamtzusammensetzung, enthält.

5. Haarstyling-Zusammensetzung nach einem der vorhergehenden Ansprüche, **dadurch gekennzeichnet, dass** sie Haarkonditionierungsmittel in einer Menge von 0,05 - 5 Gew.- %, berechnet auf die Gesamtzusammensetzung, enthält.

6. Haarstyling-Zusammensetzung nach einem der vorhergehenden Ansprüche, **dadurch gekennzeichnet, dass** sie anionische, nichtionische, kationische und/oder amphoterische oder zwitterionische Tenside als Lösungsvermittler enthält.

7. Verfahren zum Stylen von Haaren, **dadurch gekennzeichnet, dass** eine ausreichende Menge der Zusammensetzung nach einem der vorhergehenden Ansprüche auf frisch gewaschenes Haar aufgetragen wird.

8. Verfahren zur Neugestaltung von, wie in Anspruch 7 beschriebenem, gestyltem Haar, **dadurch gekennzeichnet, dass** die neue erreicht wird nach dem Befeuchten und/oder Nässen der Haare durch Besprühen und/oder Übergießen mit Wasser und/oder Wasser- Alkoholmischungen in jedem beliebigen Mischungsverhältnis, ohne das Haar neu zu waschen und/oder zu shampoonieren und ohne Verwendung eines neuen Stylingprodukts.

## Revendications

1. Composition de coiffage aqueuse et/ou hydroalcoolique avec effet rafraîchissant sur le cuir chevelu **caractérisée en ce qu'**elle comprend un polymère coiffant choisi parmi les polymères non ioniques, anioniques, cationiques et/ou amphotères ou zwitterioniques et un second polymère également choisi parmi les polymères non ioniques, anioniques, cationiques et/ou amphotères ou zwitterioniques et au moins un agent rafraîchissant choisi parmi le menthol et le lactate de menthyle, dans laquelle une composition consistant en
| | |
|---|---|
| Carbopol 934 | 0,50 |
| Polyquaternium-10 _ | 0,05 |
| Ethynol (dénaturé) | 25,00 |
| Huile de ricin hydrogénée de PEG-60 | 0,15 |
| Cétéareth-20 | 0,15 |
| Menthol naturel | 0,5 |
| Camphre naturel | 0,05 |
| Acide salicylique | 0,01 |
| Oligochitosane | 1,00 |
| Niacinamide | 0,05 |
| Vitamine E | 0,05 |
| Collagène hydrolysé | 0,01 |
| Panthénol | 0,05 |
| Ether éthylique de panthényle | 0,05 |
| Méthoxycinnamate d'octyle | 0,09 |
| Benzophénone-3 | 0,09 |
| Acétate de di-alphatocophérol | 0,03 |
| Acide lactique | 0,05 |
| Parfum | 0,35 |
| Triéthanolamine | 0,40 |
| Diméthicone | 0,05 |
| Résine MQ de 2-phénylpropyle | 0,5 |
| Eau et éléments mineurs est exclue de la portée. | qsp 100 |

2. Composition de coiffage selon la revendication 1 **caractérisée en ce qu'**elle comprend au moins un gaz propulseur.

3. Composition de coiffage selon l'une quelconque des revendications précédentes **caractérisée en ce qu'**elle comprend des polymères coiffants à une concentration de 0,05 - 10 % en poids, calculée par rapport à la composition totale.

4. Composition de coiffage selon l'une quelconque des revendications précédentes **caractérisée en ce qu'**elle comprend un agent rafraîchissant à une concentration de 0,05 - 2 % en poids, calculée par rapport à la composition totale.

5. Composition de coiffage selon l'une quelconque des revendications précédentes **caractérisée en ce qu'**elle comprend des agents de conditionnement des cheveux à une concentration de 0,05 - 5 % en poids, calculée par rapport à la composition totale.

6. Composition de coiffage selon l'une quelconque des revendications précédentes **caractérisée en ce qu'**elle comprend des tensioactifs anioniques, non ioniques, cationiques et/ou amphotères ou zwitterioniques comme solubilisants.

7. Procédé pour coiffer des cheveux **caractérisé en ce que,** sur des cheveux fraîchement lavés, est appliquée une quantité suffisante de la composition selon l'une quelconque des revendications précédentes.

8. Procédé pour recoiffer des cheveux coiffés comme dans la revendication 7 **caractérisé en ce qu'**une nouvelle coiffure est obtenue après avoir humidifié et/ou mouillé lesdits cheveux en pulvérisant et/ou en versant de l'eau et/ou un mélange eau-alcool à n'importe quel rapport sans laver fraîchement et/ou shampouiner les cheveux et sans avoir besoin d'un nouveau produit coiffant.
